(19) Europäisches Patentamt
European Patent Office
Office européen des brevets

(11) EP 0 508 900 B1

(12) # FASCICULE DE BREVET EUROPEEN

(45) Date de publication et mention de la délivrance du brevet:
**04.11.1998 Bulletin 1998/45**

(51) Int Cl.[6]: **C07J 71/00**, C07J 5/00

(21) Numéro de dépôt: **92401002.8**

(22) Date de dépôt: **09.04.1992**

(54) **Nouveaux dérivés stéroides de la pregna-1,4-dièn-3,20-dione, leur préparation, leur application à la préparation de dérivés 16,17-méthylène dioxy substitués et nouveaux intermediaires**

Neue Steroidderivate vom Typ Pregna-1,4-diene-3,20-dion, ihre Herstellung und Verwendung zur Herstellung der substituierten 16,17-Methylendioxyderivate und neuen Zwischenprodukten

New steroid derivatives of Pregna-1,4-dien-3,20-dione, their preparation and use in the preparation of substituted 16,17-methylenedioxy derivatives and new intermediates

(84) Etats contractants désignés:
**CH DE FR GB IT LI NL SE**

(30) Priorité: **10.04.1991 FR 9104339**

(43) Date de publication de la demande:
**14.10.1992 Bulletin 1992/42**

(73) Titulaire: **HOECHST MARION ROUSSEL**
**92800 Puteaux (FR)**

(72) Inventeurs:
- **Devocelle, Luc**
  **F-95210 Saint Gratien (FR)**
- **Mackiewicz, Philippe**
  **F-93190 Livry Gargan (FR)**

(74) Mandataire: **Vieillefosse, Jean-Claude et al**
**Hoechst Marion Roussel**
**Département des Brevets**
**102, Route de Noisy**
**93235 Romainville Cédex (FR)**

(56) Documents cités:

| | |
|---|---|
| **EP-A- 0 170 642** | **EP-A- 0 262 108** |
| **DE-A- 2 323 215** | **DE-A- 2 360 444** |
| **GB-A- 1 503 805** | **US-A- 4 124 707** |

- **ACTA PHARMACEUTICA SUECICA vol. 21, no. 2, 1984, pages 109 - 124; A. THALEN ET AL: 'Synthesis and Pharmacological Properties of Some 16,17-alpha-Acetals of 16-alpha-Hydroxyhydrocortisone, 16-alpha-Hydroxyprednisolone and Fluorinated 16-alpha-Hydroxyprednisolones'**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 508 900 B1

## Description

La présente invention a pour objet la préparation de stéroïdes dérivés de la pregna-1,4-diène-3,20-dione et des intermédiaires.

L'invention a ainsi pour objet un procédé de préparation du composé de formule (V) :

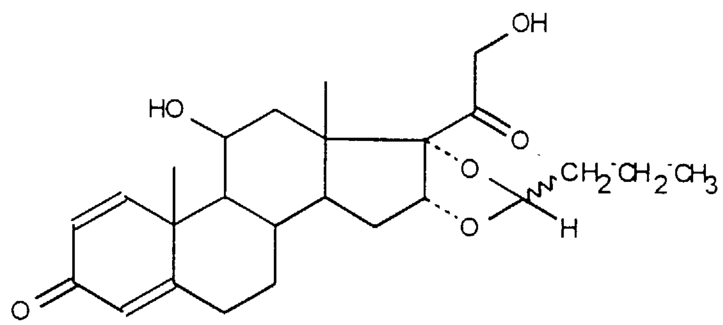

(V)

dans laquelle le trait ondulé indique l'existence de deux isomères, le composé se présentant sous forme d'un mélange de ces isomères, caractérisé en ce que l'on traite un composé de formule générale (II) :

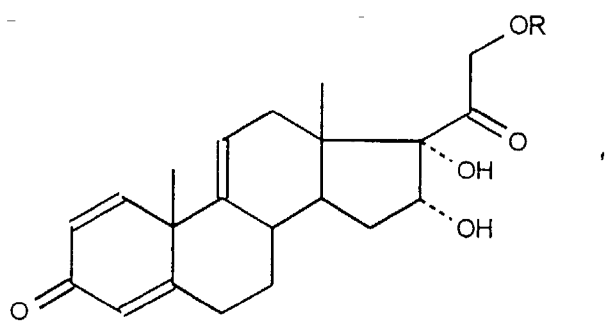

(II)

dans laquelle R représente un atome d'hydrogène, un radical acyle renfermant de 1 à 8 atomes de carbone, un radical alkoxycarbonyle ou aralkoxycarbonyle renfermant de 2 à 8 atomes de carbone ou un radical alkyl ou aryl sulfonyle renfermant au plus 8 atomes de carbone, par un agent de chloration ou de bromation et un agent de formyloxylation, en milieu acide, pour obtenir un composé de formule générale (III) :

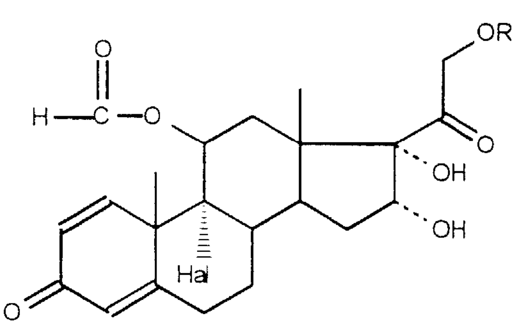

(III)

dans laquelle R est défini comme précédemment et Hal représente un atome de chlore ou de brome, que l'on traite par l'acétone en présence d'un acide fort, pour obtenir un composé de formule générale (IV) :

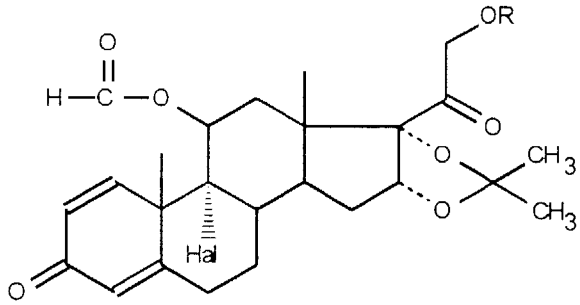

(IV)

que l'on traite par un agent de déshalogénation, pour obtenir un composé de formule générale (I) :

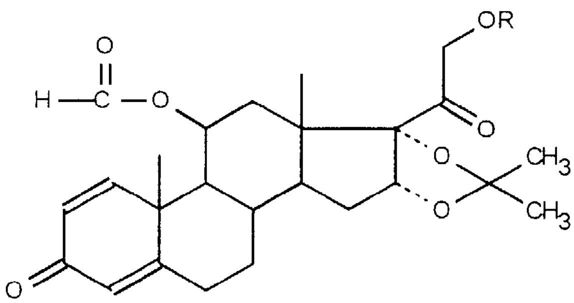

(I)

dans laquelle R est défini comme précédemment, que l'on traite par le butanal en présence d'un catalyseur acide fort, pour obtenir un composé de formule (VI) :

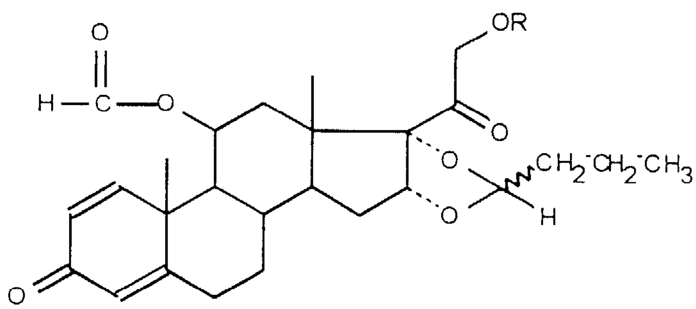

(VI)

dans laquelle R et le trait ondulé sont définis comme précédemment, que l'on soumet à une solvolyse en milieu basique, pour obtenir le composé de formule (V) attendu.

Lorsque R représente un radical acyle, il peut être notamment un radical formyle, acétyle, propionyle, butyryle, pivaloyle ou succinyle.

Lorsque R représente un radical alkoxycarbonyle ou aralkoxycarbonyle, il peut être notamment un radical benzyloxycarbonyle, allyloxycarbonyle, tert-butoxycarbonyle, éthoxycarbonyle ou méthoxycarbonyle.

Lorsque R représente un radical alkylsulfonyle, il peut être notamment un radical méthylsulfonyle.

Lorsque R représente un radical arylsulfonyle, il peut être notamment un radical phénylsulfonyle ou tolylsulfonyle.

L'invention a particulièrement pour objet un procédé tel que défini plus haut, caractérisé en ce que R représente un radical acétyle.

L'agent de chloration ou de bromation est un N-chloro ou N-bromo amide et plus particulièrement le N-chloro ou N-bromo acétamide, succinimide ou phtalimide ou la N,N-dichloro ou N,N-dibromodiméthyl hydantoïne. Il peut encore être un hypochlorite ou un hypobromite notamment de tert-butyle.

L'agent de formyloxylation est de préférence le diméthylformamide ou l'acide formique.

Comme indiqué plus haut, on opère en milieu acide. Ainsi, si on utilise le diméthylformamide, on opère de préférence en présence d'un acide fort tel que l'acide perchlorique ou l'acide fluoroborique. Si on utilise l'acide formique, on l'utilise soit seul, soit en milieu tamponné, en présence d'un formiate de sodium ou de potassium et, le cas échéant, en y ajoutant un acide tel que l'acide chlorhydrique.

L'acide fort utilisé lors de la cétalisation peut être par exemple l'acide perchlorique, l'acide fluoroborique ou encore un acide sulfonique, par exemple l'acide méthane sulfonique, l'acide benzène sulfonique ou l'acide paratoluène sulfonique, ou encore l'acide sulfurique, l'acide chlorhydrique ou l'acide bromhydrique. L'acide perchlorique ou l'acide fluoroborique sont particulièrement préférés.

On peut effectuer la cétalisation en présence d'un agent déshydratant, notamment en présence d'acétate d'isopropényle. On peut encore utiliser le diméthoxypropane, l'orthoformiate de méthyle ou d'éthyle ou un tamis moléculaire.

La déshalogénation peut être effectuée par un hydrure d'organoétain tel que l'hydrure de tributylétain, de préférence en présence d'un initiateur de réaction radicalaire, par exemple l'azobisisobutyronitrile ou le péroxyde de benzoyle. On opère de préférence au sein d'un alcanol, par exemple l'isopropanol ou le tert-butanol. On peut encore opérer dans l'acétate d'éthyle, l'acétonitrile, le toluène ou le diméthylformamide.

La déshalogénation peut encore être effectuée par l'étain (0) ou le plomb (0), ou par un sel d'étain (II) ou de plomb (II), en présence d'un donneur d'hydrogène, par exemple un thiol tel que l'éthanedithiol, le propane 1,3-dithiol ou le butane thiol, l'acide mercaptoacétique, l'acide 3-mercapto propionique ou encore l'acide hypophosphoreux, et d'un initiateur de réaction radicalaire, tel que ceux mentionnés ci-dessus, dans les conditions notamment de solvant, décrites dans la demande de brevet WO 90/09394. On peut encore utiliser un dérivé du chrome (II), notamment un sel ou un carboxylate de chrome (II), par exemple l'acétate ou le stéarate, en présence d'un donneur d'hydrogène tel que ceux mentionnés plus haut, au sein d'un solvant polaire tel que le diméthylformamide, le diméthylsulfoxyde ou le diméthyl acétamide. Enfin, on peut encore utiliser le tris (triméthylsilyl) silane, en présence d'un initiateur de réaction radicalaire tel que ceux mentionnés ci-dessus.

Le catalyseur acide fort utilisé dans la réaction avec le butanal est de préférence l'acide perchlorique. On peut encore utiliser l'acide fluoroborique. On opère dans un solvant halogéné, notamment le chlorure de méthylène ou le chloroforme, de préférence à une température comprise entre -5 et +10°C.

La solvolyse des composés de formule (VI) est de préférence une alcoolyse effectuée en présence d'une quantité catalytique de soude ou de potasse. L'alcool est de préférence le méthanol ou l'éthanol. On opère au sein d'un cosolvant qui peut être le dioxanne, le tétrahydrofuranne, ou encore le chlorure de méthylène, le chloroforme ou l'acétate d'éthyle. Le dioxanne est particulièrement préféré.

La solvolyse des composés de formule (VI) peut encore être une hydrolyse mettant en oeuvre par exemple, les bases mentionnées ci-dessus.

Selon l'invention, il est encore possible de préparer le composé de formule (V) telle que définie précédemment, par un procédé caractérisé en ce que l'on traite un composé de formule générale (II) :

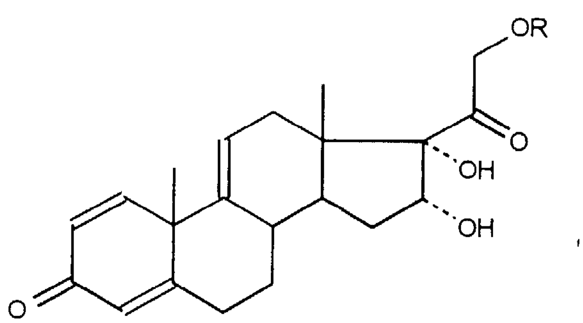

(II)

dans laquelle R représente un atome d'hydrogène, un radical acyle renfermant de 1 à 8 atomes de carbone, un radical alkoxycarbonyle ou aralkoxycarbonyle renfermant de 2 à 8 atomes de carbone ou un radical alkyl ou aryl sulfonyle renfermant au plus 8 atomes de carbone, par un agent de chloration ou de bromation et un agent de formyloxylation, en milieu acide, pour obtenir un composé de formule générale (III) :

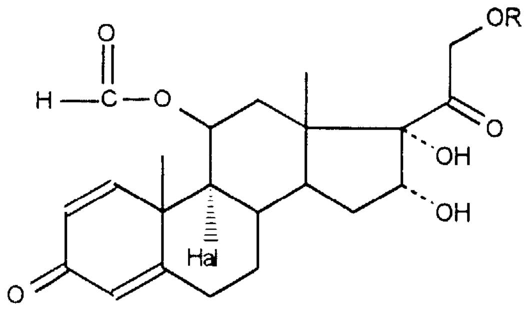

(III)

dans laquelle R est défini comme précédemment et Hal représente un atome de chlore ou de brome, que l'on traite par l'acétone en présence d'un acide fort, pour obtenir un composé de formule générale (IV) :

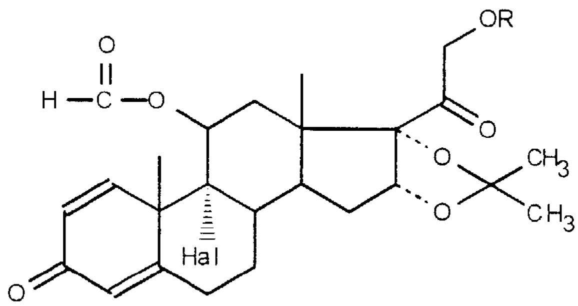

(IV)

que l'on traite par le butanal en présence d'un catalyseur acide fort, pour obtenir un composé de formule (VII) :

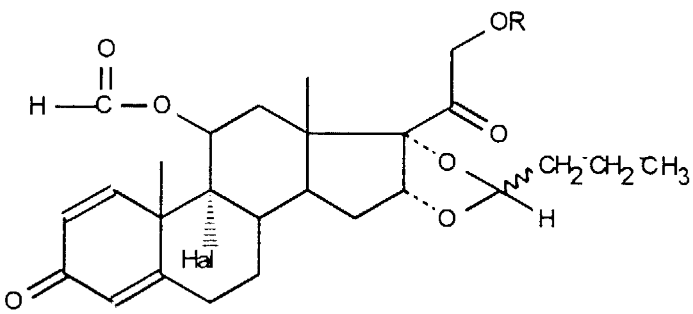

(VII)

dans laquelle Hal, R et le trait ondulé sont définis comme précédemment, que l'on traite par un agent de déshalogénation pour obtenir un composé de formule (VI) :

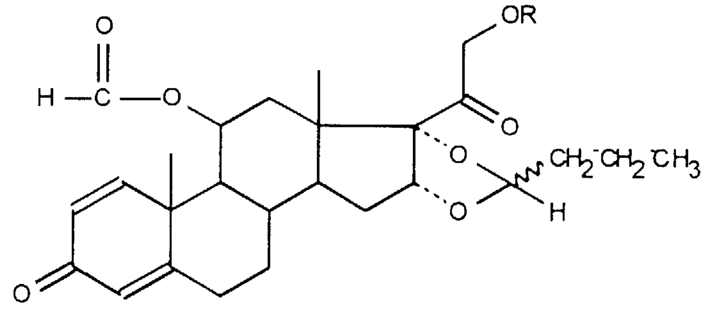

(VI)

dans laquelle R et le trait ondulé sont définis comme précédemment, que l'on soumet à une solvolyse en milieu basique, pour obtenir le composé de formule (V) attendu.

Les conditions de la mise en oeuvre de ce procédé sont les mêmes que celles qui ont été décrites plus haut pour les réaction correspondantes.

L'invention a plus particulièrement pour objet un procédé tel que défini plus haut, caractérisé en ce que R représente un radical acétyle.

L'invention a encore pour objet un procédé de préparation du composé de formule (VIII) :

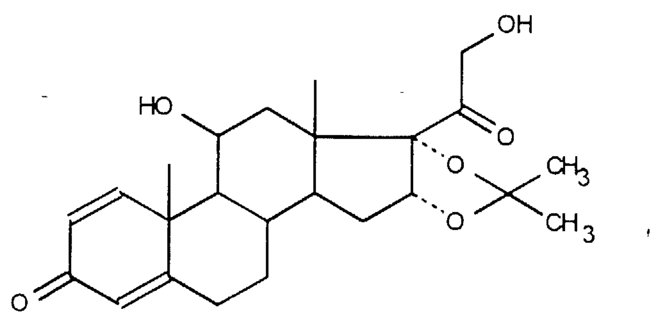

(VIII)

caractérisée en ce que l'on traite un composé de formule générale (II) :

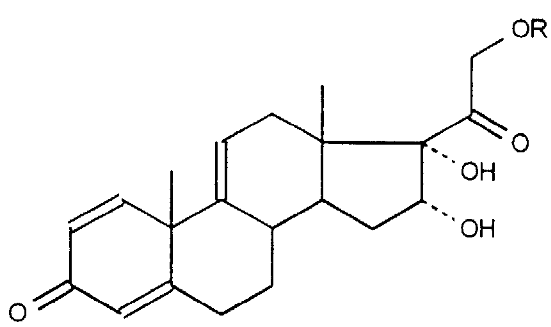

(II)

dans laquelle R représente un atome d'hydrogène, un radical acyle renfermant de 1 à 8 atomes de carbone, un radical alkoxycarbonyle ou aralkoxycarbonyle renfermant de 2 à 8 atomes de carbone ou un radical alkyl ou aryl sulfonyle renfermant au plus 8 atomes de carbone, par un agent de chloration ou de bromation et un agent de formyloxylation, en milieu acide, pour obtenir un composé de formule générale (III) :

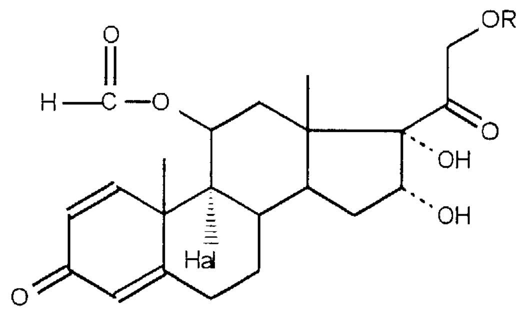

(III)

dans laquelle R est défini comme précédemment et Hal représente un atome de chlore ou de brome, que l'on traite par l'acétone en présence d'un acide fort, pour obtenir un composé de formule générale (IV) :

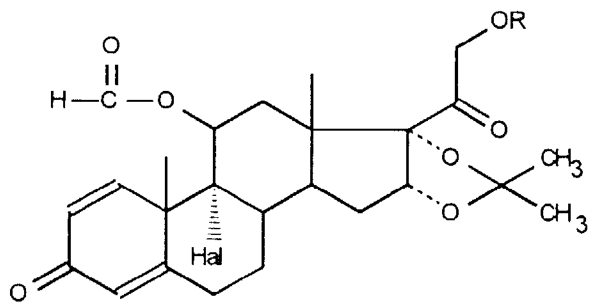

(IV)

que l'on traite par un agent de déshalogénation, pour obtenir un composé de formule générale (I) :

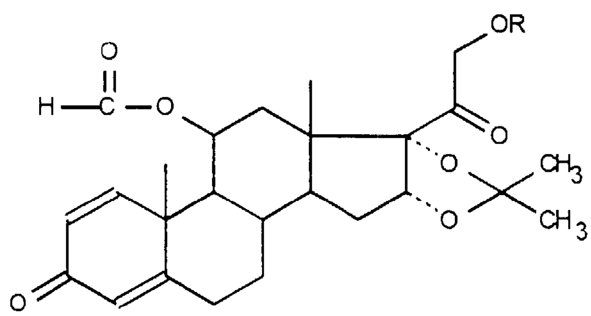

(I)

dans laquelle R est défini comme précédemment, que l'on soumet à une solvolyse en milieu basique, pour obtenir le composé de formule (VIII) attendu.

L'invention a plus particulièrement pour objet un procédé tel que défini ci-dessus, caractérisé en ce que R représente un radical acétyle.

Les conditions de la mise en oeuvre de ce procédé sont les mêmes que celles qui ont été décrites plus haut pour les réactions correspondantes.

L'invention a enfin pour objet, à titre de composés industriels nouveaux, les composés de formule (III) :

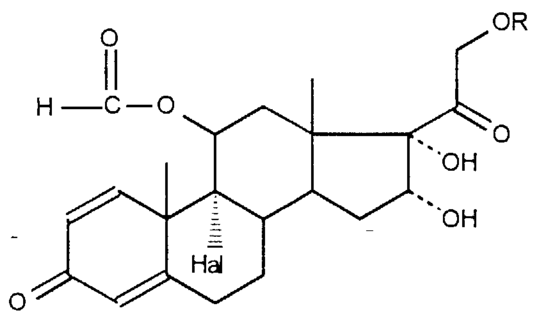

(III)

dans laquelle Hal et R sont définis comme précédemment, ainsi que les composés de formule (IV) :

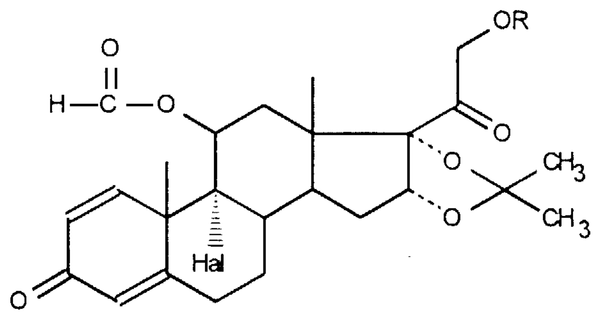

(IV)

dans laquelle Hal et R sont définis comme précédemment, les composés de formule (I) :

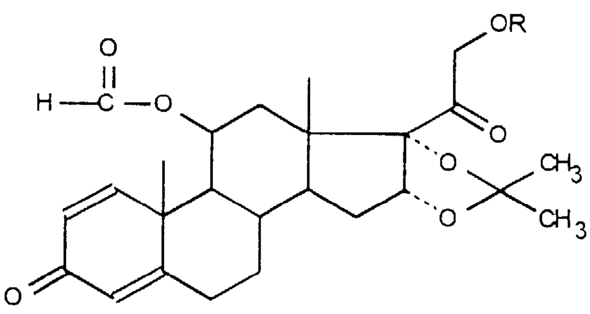

(I)

danslaquelle R représente un radical alkoxycarbonyle ou aralkoxycarbonyle renfermant de 2 à 8 atomes de carbone ou un radical alkyl ou arylsulfonyle renfermant au plus 8 atomes de carbone, les composés de formule (VI) :

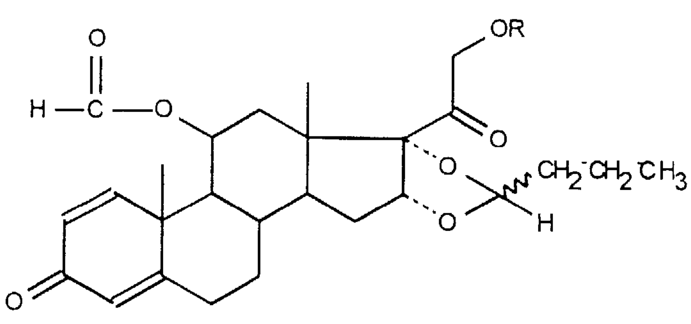

(VI)

dans laquelle R et le trait ondulé sont définis comme précédemment et les composés de formule (VII) :

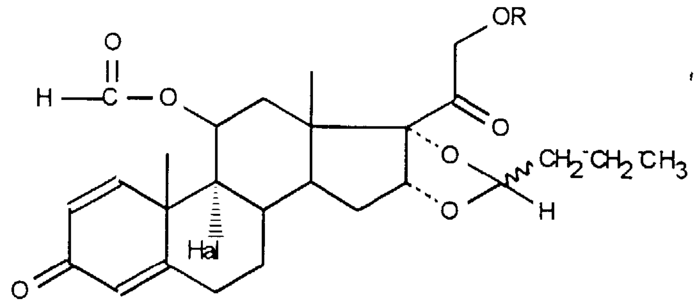

(VII)

dans laquelle Hal, R et le trait ondulé sont définis comme précédemment.

Les composés de formules (V) et (VIII) sont des composés thérapeutiquement actifs connus dans la littérature, sous les appellations budésonide et désonide, notamment dans les documents FR-A-2185405 et US n° 2990401.

Ces documents FR-A-2185405 et USP 2990401 ainsi que EP-A-164636 décrivent des synthèses qui toutes mettent en oeuvre un intermédiaire de type 11β,16α,17α-trihydroxy ou 11β-hydroxy 16,17-acétonide. Les synthèses décrites dans la présente demande s'en distinguent par le fait qu'elles mettent en oeuvre un dérivé 16α,17α-dihydroxy Δ 9(11) qui est d'emblée transformé en un dérivé 11β-hydroxy protégé sous forme de formyl bromhydrine ou chlorhydrine.

En outre, le document DE-A-2360444 décrit des 16α,17α-isopropylidendioxy 11β-formyloxy 21-hydroxy et formyloxy pregna 1,4-dièn-3,20-dione voisins des composés de formule (I).

Les composés de formule (II) utilisés au départ du procédé de l'invention sont décrits par exemple dans les brevets US 2998433, US 3047468 ou peuvent être obtenus à partir d'intermédiaires décrits dans ces brevets par des méthodes connues de l'homme du métier.

Les exemples suivants illustrent l'invention sans toutefois la limiter.

**EXEMPLE 1 : (11béta, 16alpha) 21-(acetyloxy) 11-(formyloxy) 16,17-[(1-méthyléthylidène) bis(oxy)] pregna-1,4-diène-3,20-dione**

STADE A : (9alpha, 11béta, 16alpha) 21-(acétyloxy) 16,17-dihydroxy 9-bromo 11-(formyloxy) pregna-1,4-diène-3,20-dione

On mélange à température ambiante sans gaz inerte, et à l'obscurité, 80 g de (16alpha) 21-(acétyloxy) 16,17-dihydroxy pregna-1,4,9(11)-triène-3,20-dione et 480 $cm^3$ de diméthylformamide puis y ajoute sous agitation à +5/+10°C, 42,64 g de N-bromosuccinimide puis, en 30 mn environ et en laissant remonter la température à +20/+22°C, une solution de 42,6 $cm^3$ d'acide perchlorique à 55°Bé dans 107 $cm^3$ d'eau. On laisse sous agitation à température ambiante pendant 3 h 30 mn puis verse le mélange dans un mélange eau-glace. On essore, lave les cristaux à l'eau et les sèche. On obtient 102,3 g de produit attendu que l'on peut recristalliser par dissolution dans le diméthylformamide, addition d'eau, essorage et empâtage des cristaux dans le méthanol.

F ≃ 260°C (déc.) $alpha_D^{20}$ = +111°5 ± 3° (c = 1 % DMF).

STADE B : (9alpha, 11béta, 16alpha) 21-(acétyloxy) 9-bromo 11-(formyloxy) 16,17-[(1-méthyléthylidène) bis(oxy)] pregna1,4-diène-3,20-dione

On mélange à 20°C, sous gaz inerte, 200 g de produit obtenu au stade A, 75 $cm^3$ d'acétate d'isopropényle et 1 litre d'acétone puis ajoute 1 $cm^3$ d'acide perchlorique à 55°Bé. On porte au reflux pendant 3 heures, puis refroidit à 20°C et ajoute 80 $cm^3$ de triéthylamine. On concentre le mélange réactionnel sous pression réduite à 30°C, puis distille l'acétone en maintenant le volume constant par addition de 600 $cm^3$ de méthanol. On ajoute ensuite lentement à 20°C, 200 $cm^3$ d'eau, refroidit à 0/+5°C et essore les cristaux. On les lave par un mélange méthanol-eau, les sèche et obtient 211,4 g de produit attendu. F = 207,5°C.

Spectre RMN ($CDCl_3$ MHz ppm)

$18CH_3$ : 0,86 (s) ; $19CH_3$ 1,24 (s) ; gem $diCH_3$ : 1,51-1,57 (s) ; $CH_3CO$ : 2,17 (s) ; $COCH_2$-O : 4,74-4,97 (d) ; $H_{11}$ : 5,93 (tl) ; $H_4$ : 6,09 (tl) ; $H_{16}$ : 5,00 (d) ; $H_2$ : 6,34 (dd) ; $H_1$ : 6,84 (d) ; CHO : 8,13 (s).

STADE C : (11béta, 16alpha) 21-(acétyloxy) 11-(formyloxy) 16,17-[(1-méthyléthylidène) bis (oxy)] pregna-1,4-diène-3,20-dione

On mélange sous gaz inerte à 20°C, 200 g de produit obtenu au stade B et 1 litre de tert-butanol. On porte au reflux lentement, puis ajoute 0,5 g d'azobisisobutyronitrile, puis en 1 heure environ 145,4 g d'hydrure de tributylétain. On maintient le reflux pendant 3 heures puis distille le tert-butanol en maintenant le volume constant par addition de 1,5 1 de mélange méthylcyclohexane-cyclohexane. On refroidit à 3°C environ pendant 1 heure, filtre les cristaux, les lave par le mélange méthylcyclohexane-cyclohexane et les sèche. On obtient 163,5 g de produit attendu. F = 240,5°C.
Spectre RMN ($CDCl_3$ 300 MHz ppm)
$18CH_3$ : 0,85 (s) ; $19CH_3$ : 1,32 (s) ; gem diméthyl : 1,22 (s) et 1,45 (s) ; $OCOCH_3$ : 2,17 (s) ; $COCH_2$-$OCOCH_3$ : 4,71 (d,J = 18) et 4,99 (d,J = 18) ; $H_{16}$ : 5,00 (d,J = 3,5) ; $H_{11}$ : 5,72 ; $H_4$ : 6,05 (s) ; $H_2$ : 6,30 (d,d) ; $H_1$ : 6,95 (d) ; les autres CH et $CH_2$ : 1,15 à 2,6.

**EXEMPLE 2 : (11béta, 16alpha) 11,21-dihydroxy 16,17-[butylidène bis (oxy)] pregna-1,4-diène-3,20-dione**

STADE A : (11béta, 16alpha) 21-(acétyloxy) 16,17-[butylidène bis (oxy)] 11-(formyloxy) pregna-1,4-diène-3,20-dione

On mélange sous gaz inerte, à température ambiante, 200 g de produit obtenu comme décrit à l'exemple 1 et 1200 $cm^3$ de chlorure de méthylène. On y ajoute à 0 ± 1°C, 90 $cm^3$ de butanal puis, en 5 mn environ et en maintenant la température à 0 ± 1°C, 100 $cm^3$ d'acide perchlorique à 72 %. On maintient sous agitation pendant 2 h 30 mn à la même température puis neutralise le mélange par addition de 2 l d'une solution aqueuse saturée de bicarbonate de sodium et agite le mélange à 0 ± 2°C pendant 30 mn. On ajoute alors 190 g de métabisulfite de sodium, maintient sous agitation à 0 ± 2°C pendant 30 mn puis réchauffe à température ambiante, décante, extrait la phase aqueuse au chlorure de méthylène, lave les phases organiques réunies à l'eau, les sèche et les traite par 20 g de charbon actif. On évapore le chlorure de méthylène en maintenant le volume constant par ajoute de mélange cyclohexane-méthylcyclohexane, on agite la suspension 1 h à 0 ± 1°C, et obtient 200,4 g de produit attendu utilisé tel quel pour le stade suivant.
Spectre RMN ($CDCl_3$ 300 MHz ppm)
$18CH_3$ : 0,86 et 0,91 ; $19CH_3$ : 1,32 (s) ; $CH_3$ du butylidène : 0,92 (t) et 0,94 (t) ; OAc : 2,15 et 2,16 (s) ; CH $^{O}_{O}$: 4,63 (t) et 5,20 (t) ; $H_{16}$ : 4,86 (d) et 5,14 (d) ; CO-$CH_2$-O-CO : 4,71 à 4,88 ; $H_{11}$ : 5,71 (m) ; $H_4$ : 6,04 ; $H_2$ : 6,28 (d,d dédoublé) ; $H_1$ : 6,94 (d dédoublé) ; OCH-O : 8,17 (s).

STADE B : (11béta, 16alpha) 11,21-dihydroxy 16,17-[butylidène bis(oxy)] pregna-1,4-diène-3,20-dione

On mélange à température ambiante sous gaz inerte, 190 g de produit obtenu au stade A et 1140 $cm^3$ de dioxanne puis distille le solvant à 30/35°C, sous 35 à 40 mm Hg jusqu'à élimination de l'eau présente, et obtention dans le milieu réactionnel d'un volume de 1 l environ. On refroidit à 20/25°C, ajoute 950 $cm^3$ de méthanol, refroidit à 10°C ± 1°C et ajoute 92 $cm^3$ d'une solution méthanolique de potasse 0,96 M. On agite 1 heure puis ajoute, toujours à 10°C ± 1°C, 2 $cm^3$ d'acide acétique. On agite à nouveau pendant 30 mn et traite par 9,5 g de charbon actif. On verse lentement la solution dans 950 $cm^3$ d'eau portée à 65°C environ, sous pression de 35 à 40 mm Hg tout en distillant à volume constant, puis refroidit et agite la suspension obtenue pendant 2 heures à température ambiante. On essore les cristaux obtenus, on les lave à l'eau puis les sèche. On obtient 159,3 g de produit attendu. On purifie le produit dans 5 volumes d'un mélange d'eau et de méthylisobutylcétone (3/1), que l'on porte à 60°C environ, sous agitation pendant 1 heure, puis que l'on refroidit à 20°C pendant 2 heures. On essore les cristaux et les lave par de la méthylisobutylcétone puis par de l'eau, puis les sèche. On obtient finalement 137,7 g de produit attendu.
$alpha_D^{25}$ = 103,3 (1 % dans $CH_2Cl_2$).
Spectre RMN ($CDCl_3$ 300 MHz ppm)
$18CH_3$ : 0,93 et 0,99 (s) ; $19CH_3$ : 1,46 (s) ; $CH_3$ du butylidène : 0,92 (t) et 0,93 (t) ; CO-$CH_2$-O : 4,20 (d) et 4,63 (d), 4,27 (d) et 4,52 (d) ; $H_{11}$ : 4,52 (m) ; CH $^{O}_{O}$ : 4,55 (t) et 5,17 (t) ; $H_{16}$ : 4,90 (d) ; $H_4$ : 6,03 ; $H_2$ : 6,28 (d,d dédoublé) ; $H_1$ : 7,30 (d dédoublé).

**EXEMPLE 3 : (11béta, 16alpha) 11,21-dihydroxy 16,17-[(1-méthyl éthylidène) bis(oxy)] pregna-1,4-diène-3,20-dione**

On mélange à température ambiante sous gaz inerte, 140 g de produit obtenu comme décrit à l'exemple 1, 700 $cm^3$ de méthanol et 700 $cm^3$ de dioxanne. On refroidit à 10°C ± 1°C, ajoute en 5 mn, 33,5 $cm^3$ d'une solution 0,86 M de potasse méthanolique, puis maintient sous agitation à la même température pendant 1 h. On ajoute ensuite 0,8 $cm^3$ d'acide acétique puis traite par 7 g de charbon actif. La solution est ensuite versé lentement sur 700 $cm^3$ d'eau à

25°C environ, sous pression de 50 à 60 mm Hg tout en distillant à volume constant. L'addition terminée, on ajoute dans les mêmes conditions 140 $cm^3$ d'eau puis refroidit à 10°C environ et agite 1 h. On essore les cristaux, les lave à l'eau et les sèche. On obtient 118 g de produit attendu. On empâte le produit à chaud dans 590 $cm^3$ d'éthanol puis on l'essore et le sèche. On effectue une nouvelle purification en versant lentement une solution de 106 g de produit dans 423 $cm^3$ de diméthylformamide, dans 423 $cm^3$ d'eau portée à 50°C environ. On refroidit à +15°C environ et essore puis sèche les cristaux. On obtient 103,6 g de produit attendu. PF = 281°C.

Spectre RMN ($CDCl_3$ 300 MHz ppm)

18$CH_3$ : 0,89 (s) ; 21OH : 3,11 (t,J = 5 Hz) ; 21$CH_2$ : 4,17 (dd, J = 5 et 20 Hz), 4,67 (dd, J = 5 et 20 Hz) ; $H_{11}$ : 4,51 (m) ; $H_{16}$ : 5,05 (d) ; $H_4$ : 6,03 (s) ; $H_2$ : 6,28 (d,d) ; $H_1$ : 7,28 (d).

**Revendications**

1. Procédé de préparation du composé de formule (V) :

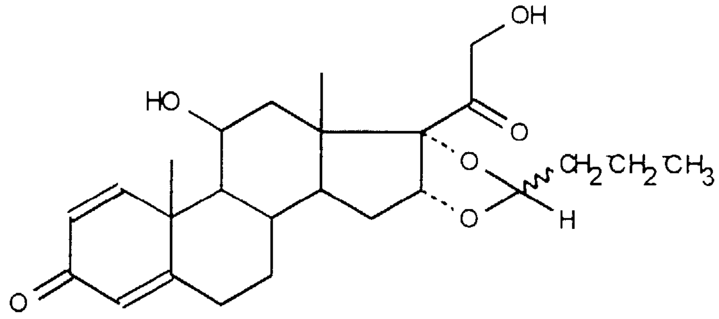

(V)

dans laquelle le trait ondulé indique l'existence de deux isomères, le composé se présentant sous forme d'un mélange de ces isomères, caractérisé en ce que l'on traite un composé de formule générale (II) :

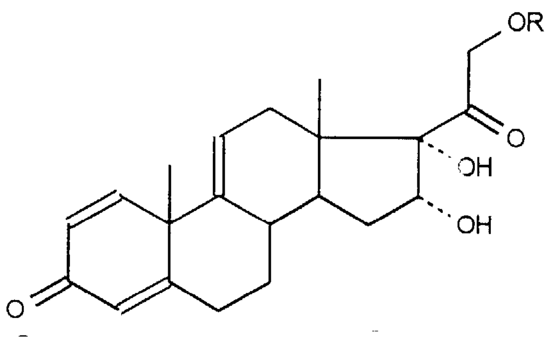

(II)

dans laquelle R représente un atome d'hydrogène, un radical acyle renfermant de 1 à 8 atomes de carbone, un radical alkoxycarbonyle ou aralkoxycarbonyle renfermant de 2 à 8 atomes de carbone ou un radical alkyl ou aryl sulfonyle renfermant au plus 8 atomes de carbone, par un agent de chloration ou de bromation et un agent de formyloxylation, en milieu acide, pour obtenir un composé de formule générale (III) :

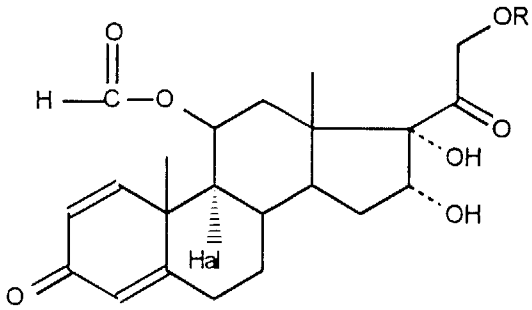

(III)

dans laquelle R est défini comme précédemment et Hal représente un atome de chlore ou de brome, que l'on traite par l'acétone en présence d'un acide fort, pour obtenir un composé de formule générale (IV) :

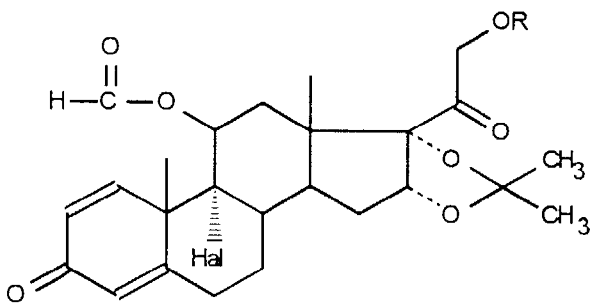

(IV)

que l'on traite par un agent de déshalogénation, pour obtenir un composé de formule générale (I) :

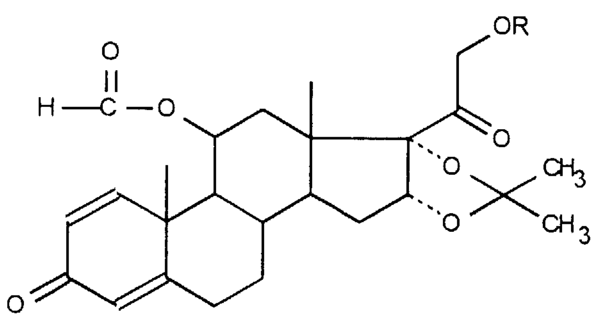

(I)

dans laquelle R est défini comme précédemment, que l'on traite par le butanal en présence d'un catalyseur acide fort, pour obtenir un composé de formule (VI) :

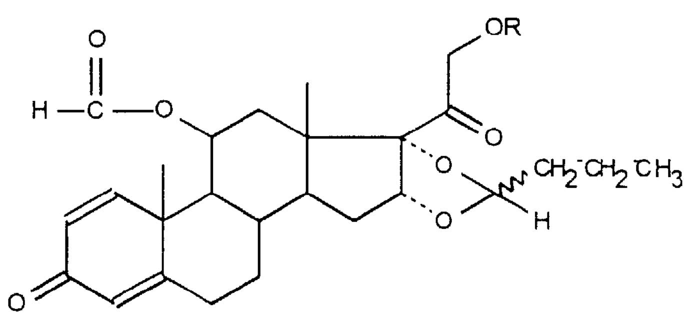

(VI)

dans laquelle R et le trait ondulé sont définis comme précédemment, que l'on soumet à une solvolyse en milieu basique, pour obtenir le composé de formule (V) attendu.

**2.** Procédé selon la revendication 1, caractérisé en ce que R représente un radical acétyle.

**3.** Procédé selon la revendication 1 ou 2, caractérisé en ce que :

- l'agent de chloration ou de bromation est choisi dans le groupe constitué par les N-chloro et N-bromo acétamides, succinimides et phtalimides, les N,N-dichloro et N,N-dibromo diméthyl hydantoïnes, l'hypochlorite de tert-butyle et l'hypobromite de tert-butyle ;
- l'agent de formyloxylation est le diméthylformamide ou l'acide formique ;
- l'acide fort utilisé lors de la cétalisation est l'acide perchlorique-ou l'acide fluoroborique ;
- la cétalisation est effectuée en présence d'un agent déshydratant ;
- la déshalogénation est effectuée par action de l'hydrure de tributylétain, en présence d'un initiateur de réaction radicalaire ;
- le catalyseur acide fort est l'acide perchlorique ;
- la solvolyse finale est une alcoolyse effectué par la soude ou la potasse méthanolique, au sein du dioxanne.

**4.** Procédé de préparation du composé de formule (V) :

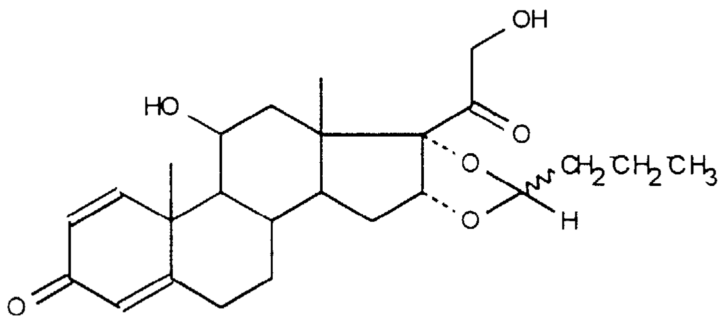

(V)

dans laquelle le trait ondulé indique l'existence de deux isomères, le composé se présentant sous forme d'un mélange de ces isomères, caractérisé en ce que l'on traite un composé de formule générale (II) :

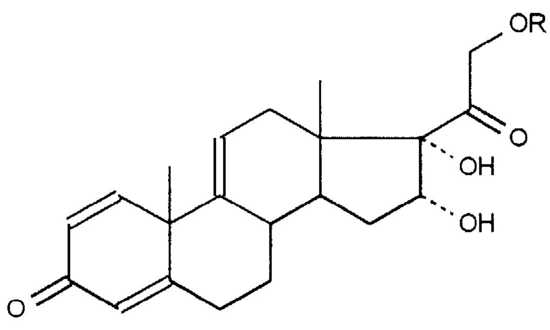

(II)

dans laquelle R représente un atome d'hydrogène, un radical acyle renfermant de 1 à 8 atomes de carbone, un radical alkoxycarbonyle ou aralkoxycarbonyle renfermant de 2 à 8 atomes de carbone ou un radical alkyl ou aryl sulfonyle renfermant au plus 8 atomes de carbone, par un agent de chloration ou de bromation et un agent de formyloxylation, en milieu acide, pour obtenir un composé de formule générale (III) :

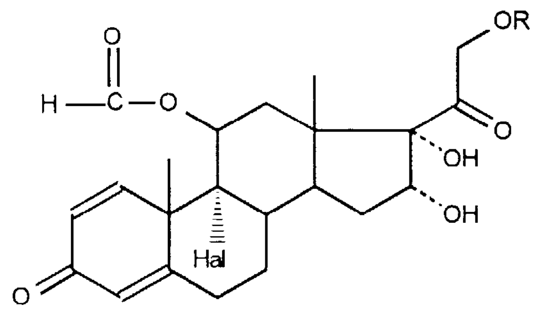

(III)

dans laquelle R est défini comme précédemment et Hal représente un atome de chlore ou de brome, que l'on traite par l'acétone en présence d'un acide fort, pour obtenir un composé de formule générale (IV) :

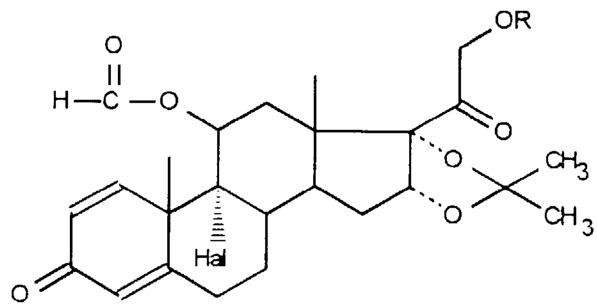

(IV)

que l'on traite par le butanal en présence d'un catalyseur acide fort, pour obtenir un composé de formule (VII) :

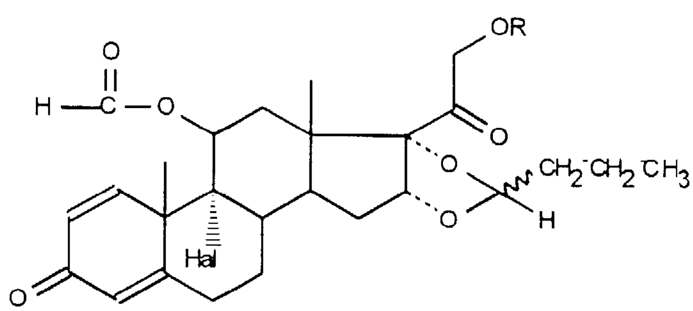

(VII)

dans laquelle Hal, R et le trait ondulé sont définis comme précédemment, que l'on traite par un agent de déshalogénation pour obtenir un composé de formule (VI) :

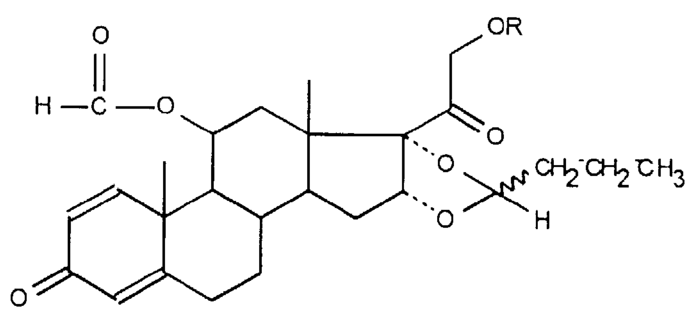

(VI)

dans laquelle R et le trait ondulé sont définis comme précédemment, que l'on soumet à une solvolyse en milieu basique, pour obtenir le composé de formule (V) attendu.

5. Procédé selon la revendication 4, caractérisé en ce que R représente un radical acétyle.

6. Procédé selon la revendication 4 ou 5, caractérisé en ce que :

- l'agent de chloration ou de bromation est choisi dans le groupe constitué par les N-chloro et N-bromo acétamides, succinimides et phtalimides, les N,N-dichloro et N,N-dibromo diméthyl hydantoines, l'hypochlorite de tert-butyle et l'hypobromite de tert-butyle ;
- l'agent de formyloxylation est le diméthylformamide ou l'acide formique ;
- l'acide fort utilisé lors de la cétalisation est l'acide perchlorique ou l'acide fluoroborique ;
- la cétalisation est effectuée en présence d'un agent déshydratant ;
- le catalyseur acide fort est l'acide perchlorique ;
- la déshalogénation est effectuée par action de l'hydrure de tributylétain, en présence d'un initiateur de réaction radicalaire ;
- la solvolyse finale est une alcoolyse effectué par la soude ou la potasse méthanolique, au sein du dioxanne.

7. Procédé de préparation du composé de formule (VIII) :

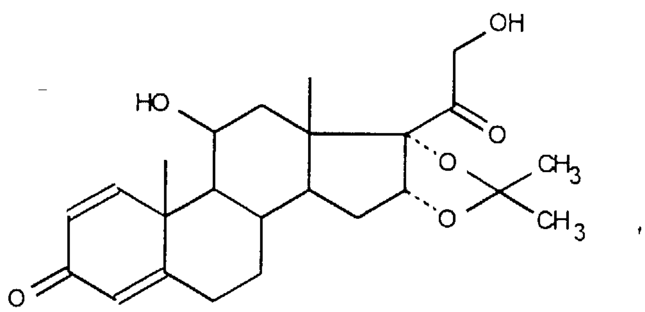

(VIII)

caractérisé en ce que l'on traite un composé de formule générale (II)

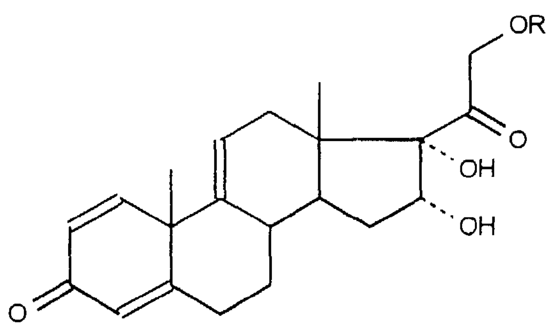

(II)

dans laquelle R représente un atome d'hydrogène, un radical acyle renfermant de 1 à 8 atomes de carbone, un radical alkoxycarbonyle ou aralkoxycarbonyle renfermant de 2 à 8 atomes de carbone ou un radical alkyl ou aryl sulfonyle renfermant au plus 8 atomes de carbone, par un agent de chloration ou de bromation et un agent de formyloxylation, en milieu acide, pour obtenir un composé de formule générale (III) :

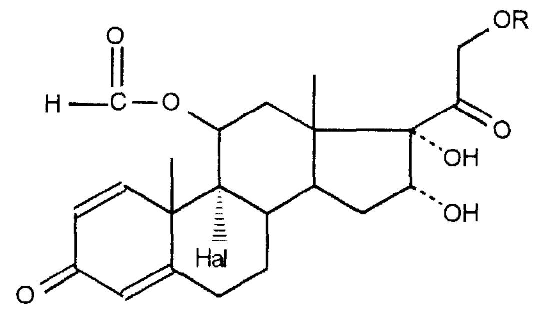

(III)

dans laquelle R est défini comme précédemment et Hal représente un atome de chlore ou de brome, que l'on traite par l'acétone en présence d'un acide fort, pour obtenir un composé de formule générale (IV) :

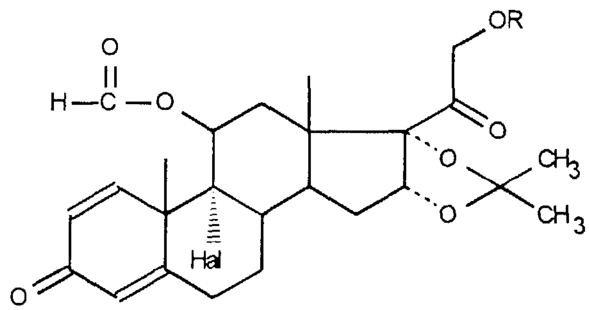

(IV)

que l'on traite par un agent de déshalogénation, pour obtenir un composé de formule générale (I) :

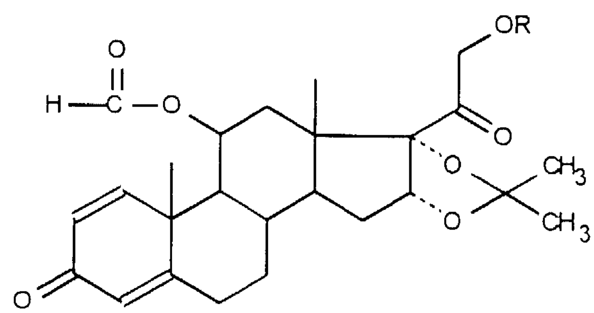

(I)

dans laquelle R est défini comme précédemment, que l'on soumet à une solvolyse en milieu basique, pour obtenir le composé de formule (VIII) attendu.

**8.** Procédé selon la revendication 7, caractérisé en ce que R représente un radical acétyle.

**9.** Procédé selon la revendication 7 ou 8, caractérisé en ce que :

- l'agent de chloration ou de bromation est choisi dans le groupe constitué par les N-chloro et N-bromo acétamides, succinimides et phtalimides, les N,N-dichloro et N,N-dibromo diméthyl hydantoînes, l'hypochlorite de tert-butyle et l'hypobromite de tert-butyle ;
- l'agent de formyloxylation est le diméthylformamide ou l'acide formique ;
- l'acide fort utilisé lors de la cétalisation est l'acide perchlorique ou l'acide fluoroborique ;
- la cétalisation est effectuée en présence d'un agent déshydratant ;
- la déshalogénation est effectuée par action de l'hydrure de tributylétain, en présence d'un initiateur de réaction

radicalaire ;
- la solvolyse finale est une alcoolyse effectué par la soude ou la potasse méthanolique, au sein du dioxanne.

**10.** A titre de composés industriels nouveaux, les composés de formule (III) :

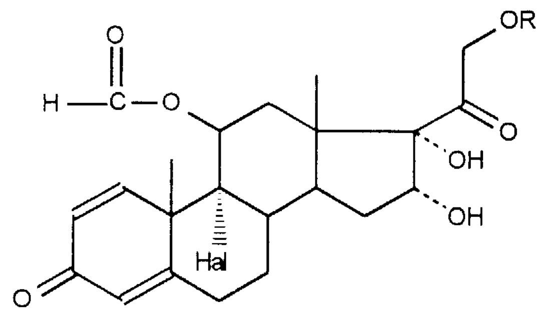

(III)

dans laquelle Hal et R sont définis comme à la revendication 1.

**11.** A titre de composés industriels nouveaux, les composés de formule (IV) :

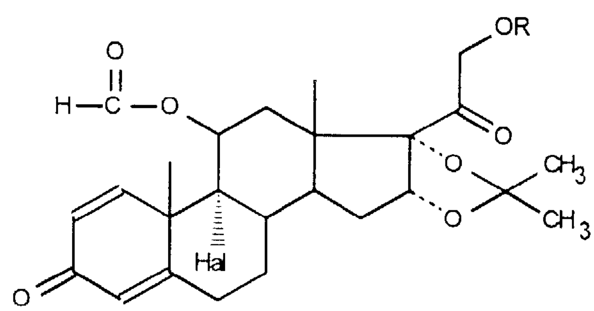

(IV)

dans laquelle Hal et R sont définis comme à la revendication 1.

**12.** A titre de composés industriels nouveaux, les composés de formule (I) :

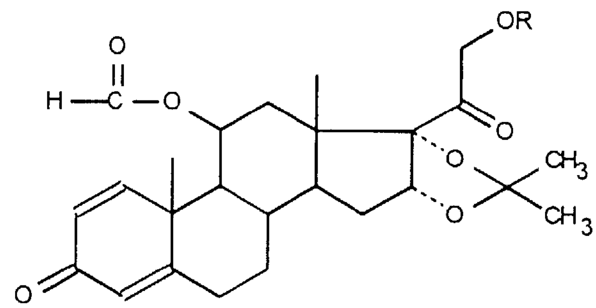

(I)

dans laquelle R représente un radical alkoxycarbonyle ou aralkoxycarbonyle renfermant de 2 à 8 atomes de carbone ou un radical alkyl ou aryl sulfonyle renfermant au plus 8 atomes de carbone.

**13.** A titre de composés industriels nouveaux, les composés de formule (VI) :

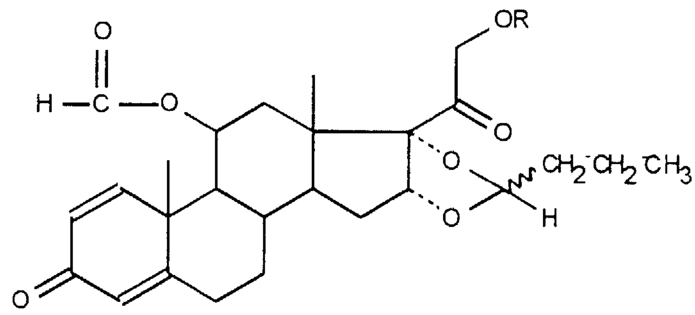

(VI)

dans laquelle R et le trait ondulé sont définis comme à la revendication 1.

**14.** A titre de composés industriels nouveaux, les composés de formule (VII) :

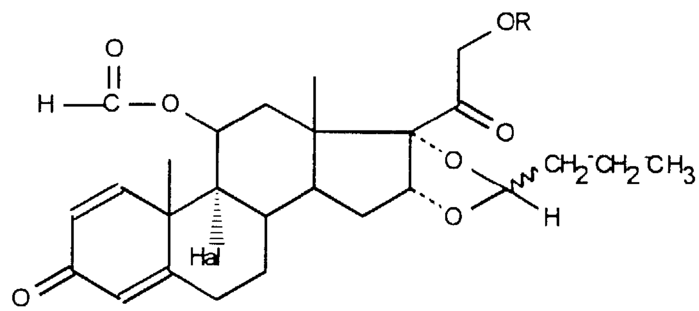

(VII)

dans laquelle Hal et R et le trait ondulé sont définis comme à la revendication 1.

**Patentansprüche**

**1.** Verfahren zur Herstellung der Verbindung der Formel (V):

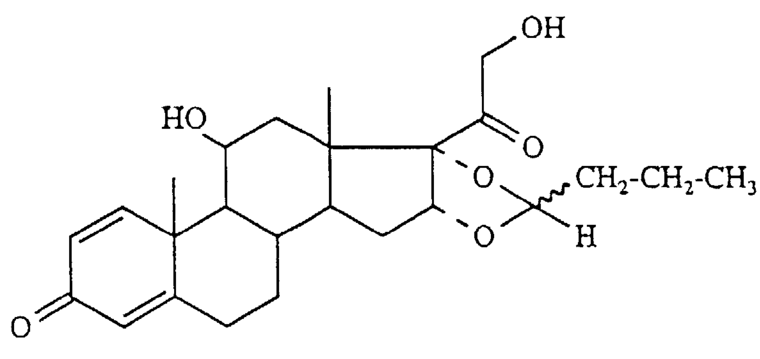

(V)

in der die wellenförmig dargestellte Bindung die Existenz zweier Isomeren veranschaulicht, wobei die Verbindung in Form eines Gemischs dieser Isomeren vorliegt, dadurch gekennzeichnet, daß eine Verbindung der allgemeinen Formel (II):

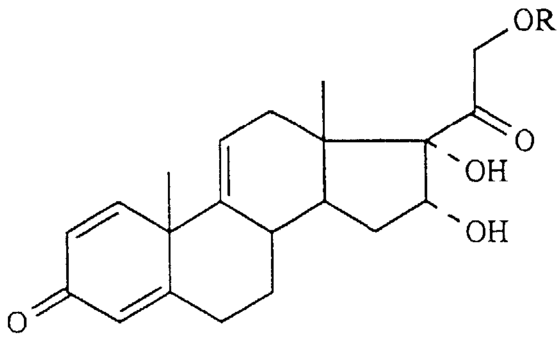

(II)

in der R ein Wasserstoffatom, einen Acylrest mit 1 bis 8 Kohlenstoffatomen, einen Alkoxycarbonyl- oder Arylalkoxycarbonylrest mit 2 bis 8 Kohlenstoffatomen oder einen Alkyl- oder Arylsulfonylrest mit maximal 8 Kohlenstoffatomen darstellt,
in saurem Milieu mit einem Chlorierungs- oder Bromierungsmittel und einem Formyloxylierungsmittel behandelt wird, um eine Verbindung der allgemeinen Formel (III) zu erhalten:

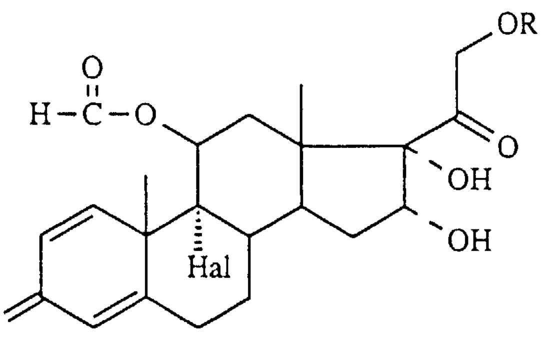

(III)

in der R die oben angegebene Bedeutung besitzt und Hal ein Chlor- oder Bromatom darstellt;
die in Gegenwart einer starken Säure mit Aceton behandelt wird, um eine Verbindung der allgemeinen Formel (IV):

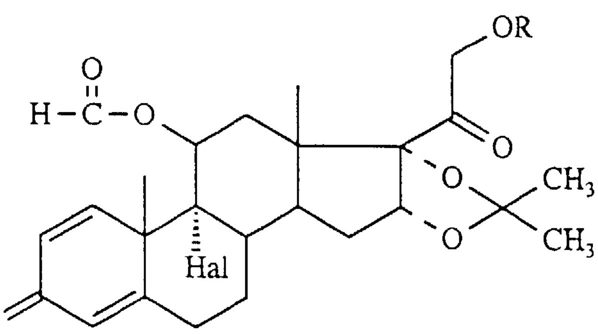

(IV)

zu erhalten, die mit einem Dehalogenierungsmittel behandelt wird, um eine Verbindung der allgemeinen Formel (I) zu erhalten:

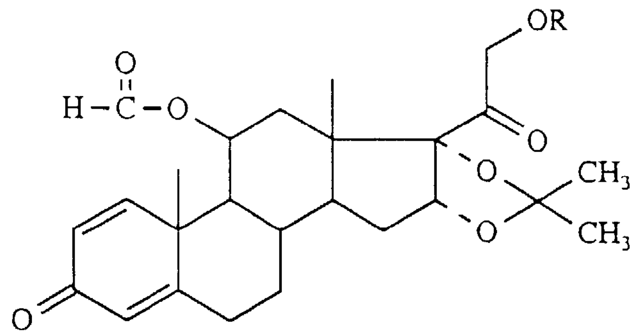

(I)

in der R die oben angegebene Bedeutung besitzt;
die in Gegenwart eines stark sauren Katalysators mit Butanal behandelt wird, um eine Verbindung der Formel (VI) zu erhalten:

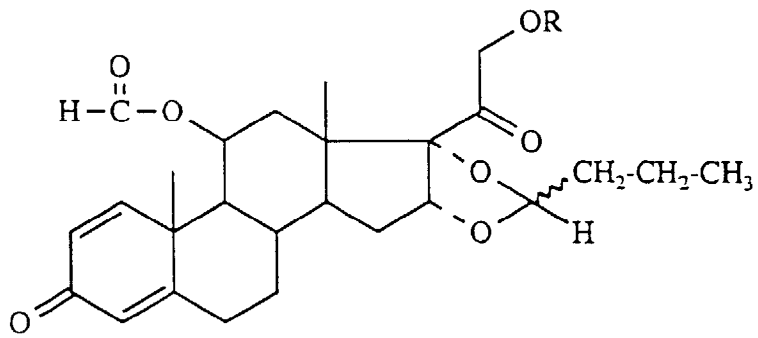

(VI)

in der R und die wellenförmig dargestellte Bindung die oben angegebene Bedeutung besitzen;
die in basischem Milieu einer Solvolyse unterworfen wird, um die gesuchte Verbindung der Formel (V) zu erhalten.

**2.** Verfahren nach Anspruch 1, dadurch gekennzeichnet, daß R einen Acetylrest darstellt.

**3.** Verfahren nach Anspruch 1 oder 2, dadurch gekennzeichnet, daß:

- das Chlorierungs- beziehungsweise Bromierungsmittel unter den N-Chlor- und N-Bromacetamiden, den N-Chlor- und N-Bromsuccinimiden und den N-Chlor- und N-Bromphthalimiden, den N,N-Dichlor- und N,N-Dibromdimethylhydantoinen, dem tert-Butylhypochlorit und dem tert-Butylhypobromit ausgewählt ist;
- das Formyloxylierungsmittel Dimethylformamid oder Ameisensäure ist;
- die für die Ketalisierung verwendete starke Säure Perchlorsäure oder Borfluorwasserstoffsäure ist;
- die Ketalisierung in Gegenwart eines Dehydratisierungsmittels durchgeführt wird;
- die Dehalogenierung durch Einwirkung von Tributylzinnhydrid in Gegenwart eines Initiators der Radikalreaktion erfolgt;
- der stark saure Katalysator Perchlorsäure ist und
- die abschließende Solvolyse eine in Dioxan mit methanolischer Natronlauge oder Kalilauge durchgeführte Alkoholyse ist.

**4.** Verfahren zur Herstellung der Verbindung der Formel (V):

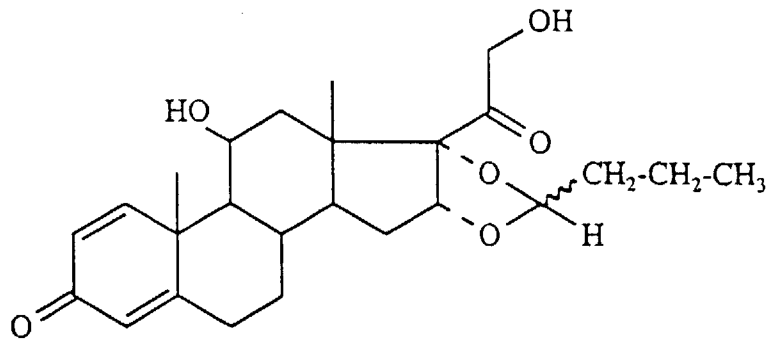

(V)

in der die wellenförmig dargestellte Bindung die Existenz zweier Isomeren veranschaulicht,
wobei die Verbindung in Form eines Gemischs dieser Isomeren vorliegt, dadurch gekennzeichnet, daß eine Verbindung der allgemeinen Formel (II):

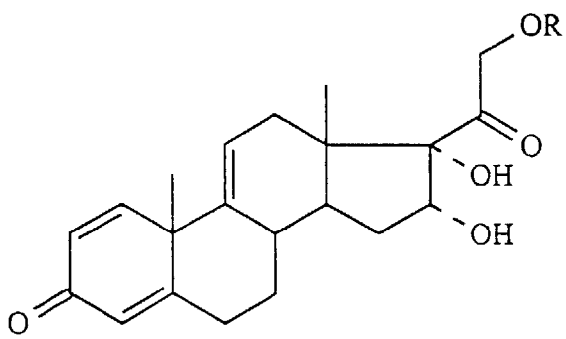

(II)

in der R ein Wasserstoffatom, einen Acylrest mit 1 bis 8 Kohlenstoffatomen, einen Alkoxycarbonyl- oder Arylalkoxycarbonylrest mit 2 bis 8 Kohlenstoffatomen oder einen Alkyl- oder Arylsulfonylrest mit maximal 8 Kohlenstoffatomen darstellt,
in saurem Milieu mit einem Chlorierungs- oder Bromierungsmittel und einem Formyloxylierungsmittel behandelt wird, um eine Verbindung der allgemeinen Formel (III) zu erhalten:

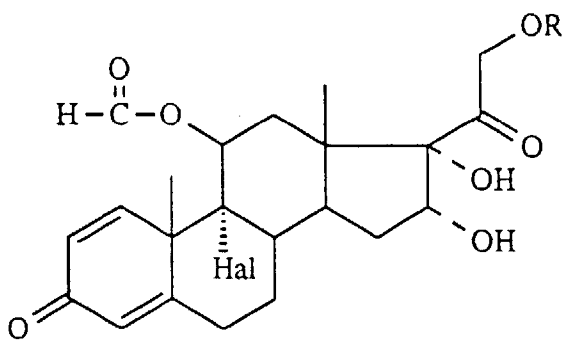

(III)

in der R die oben angegebene Bedeutung besitzt und Hal ein Chlor- oder Bromatom darstellt;
die in Gegenwart einer starken Säure mit Aceton behandelt wird, um eine Verbindung der allgemeinen Formel (IV):

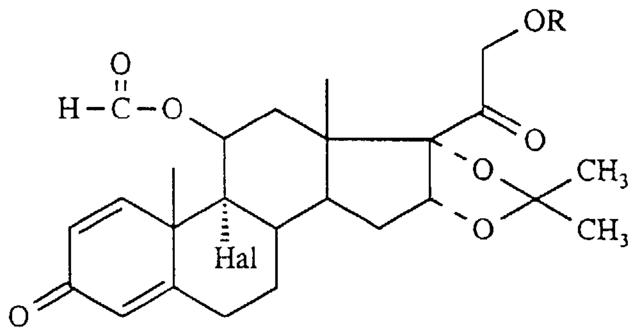

(IV)

zu erhalten, die in Gegenwart eines stark sauren Katalysators mit Butanal behandelt wird, um eine Verbindung der Formel (VII) zu erhalten:

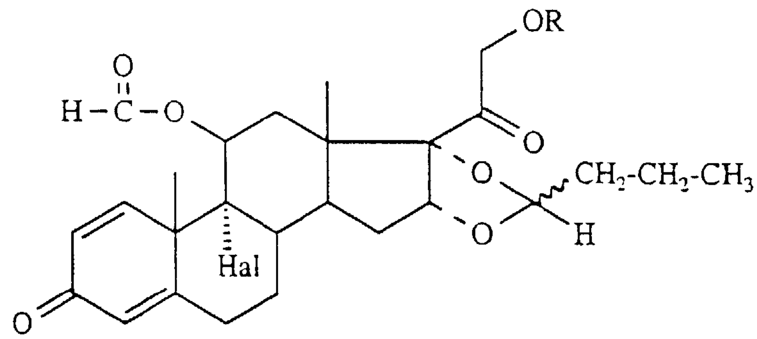

(VII)

in der Hal, R und die wellenförmig dargestellte Bindung die oben angegebene Bedeutung besitzen;
die mit einem Dehalogenierungsmittel behandelt wird, um eine Verbindung der Formel (VI) zu erhalten:

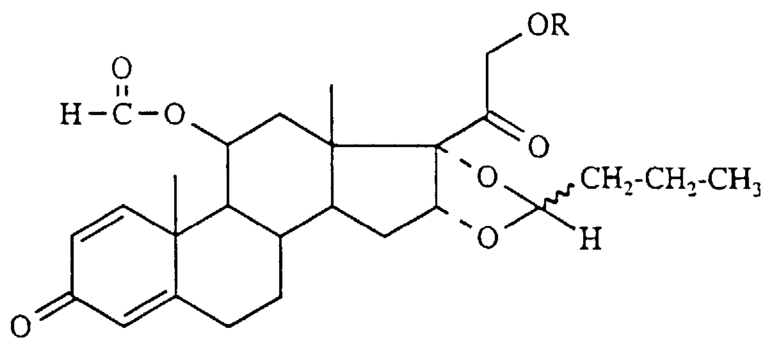

(VI)

in der R und die wellenförmig dargestellte Bindung die oben angegebene Bedeutung besitzen;
die in basischem Milieu einer Solvolyse unterworfen wird, um die gesuchte Verbindung der Formel (V) zu erhalten.

**5.** Verfahren nach Anspruch 4, dadurch gekennzeichnet, daß R einen Acetylrest darstellt.

**6.** Verfahren nach Anspruch 4 oder 5, dadurch gekennzeichnet, daß:

- das Chlorierungs- beziehungsweise Bromierungsmittel unter den N-Chlor- und N-Bromacetamiden, den N-Chlor- und N-Bromsuccinimiden und den N-Chlor- und N-Bromphthalimiden, den N,N-Dichlor- und N,N-Dibromdimethylhydantoinen, dem tert-Butylhypochlorit und dem tert-Butylhypobromit ausgewählt ist;
- das Formyloxylierungsmittel Dimethylformamid oder Ameisensäure ist;
- die für die Ketalisierung verwendete starke Säure Perchlorsäure oder Borfluorwasserstoffsäure ist;

- die Ketalisierung in Gegenwart eines Dehydratisierungsmittels durchgeführt wird;
- der stark saure Katalysator Perchlorsäure ist;
- die Dehalogenierung durch Einwirkung von Tributylzinnhydrid in Gegenwart eines Initiators der Radikalreaktion erfolgt
  und
- die abschließende Solvolyse eine in Dioxan mit methanolischer Natronlauge oder Kalilauge durchgeführte Alkoholyse ist.

**7.** Verfahren zur Herstellung der Verbindung der Formel (VIII):

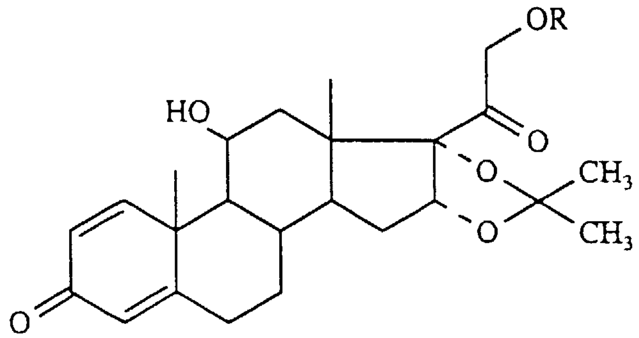

(VIII)

dadurch gekennzeichnet, daß eine Verbindung der allgemeinen Formel (II):

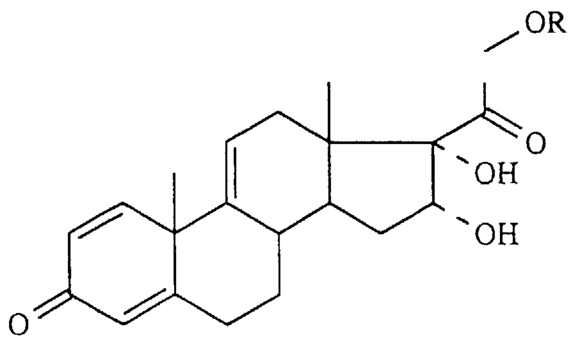

(II)

in der R ein Wasserstoffatom, einen Acylrest mit 1 bis 8 Kohlenstoffatomen, einen Alkoxycarbonyl- oder Arylalkoxycarbonylrest mit 2 bis 8 Kohlenstoffatomen oder einen Alkyl- oder Arylsulfonylrest mit maximal 8 Kohlenstoffatomen darstellt,
in saurem Milieu mit einem Chlorierungs- oder Bromierungsmittel und einem Formyloxylierungsmittel behandelt wird, um eine Verbindung der allgemeinen Formel (III) zu erhalten:

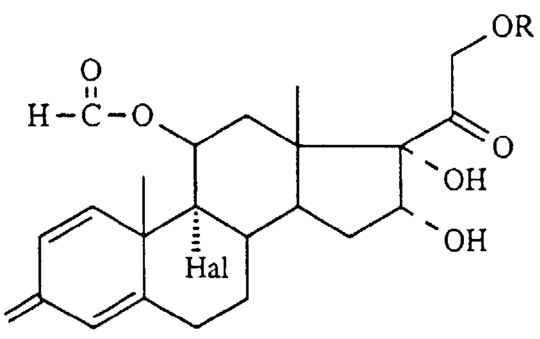

(III)

in der R die oben angegebene Bedeutung besitzt und Hal ein Chlor- oder Bromatom darstellt;
die in Gegenwart einer starken Säure mit Aceton behandelt wird, um eine Verbindung der allgemeinen Formel (IV):

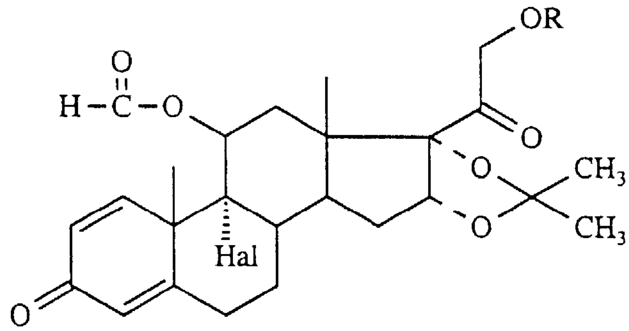

(IV)

zu erhalten, die mit einem Dehalogenierungsmittel behandelt wird, um eine Verbindung der allgemeinen Formel (I) zu erhalten:

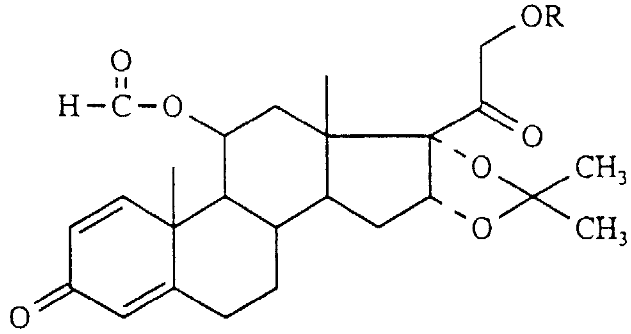

(I)

in der R die oben angegebene Bedeutung besitzt;
die in basischem Milieu einer Solvolyse unterworfen wird, um die gesuchte Verbindung der Formel (VIII) zu erhalten.

**8.** Verfahren nach Anspruch 7, dadurch gekennzeichnet, daß R einen Acetylrest darstellt.

**9.** Verfahren nach Anspruch 7 oder 8, dadurch gekennzeichnet, daß:

- das Chlorierungs- beziehungsweise Bromierungsmittel unter den N-Chlor- und N-Bromacetamiden, den N-Chlor- und N-Bromsuccinimiden und den N-Chlor- und N-Bromphthalimiden, den N,N-Dichlor- und N,N-Dibromdimethylhydantoinen, dem tert-Butylhypochlorit und dem tert-Butylhypobromit ausgewählt ist;
- das Formyloxylierungsmittel Dimethylformamid oder Ameisensäure ist;
- die für die Ketalisierung verwendete starke Säure Perchlorsäure oder Borfluorwasserstoffsäure ist;
- die Ketalisierung in Gegenwart eines Dehydratisierungsmittels durchgeführt wird;
- die Dehalogenierung durch Einwirkung von Tributylzinnhydrid in Gegenwart eines Initiators der Radikalreaktion erfolgt
  und
- die abschließende Solvolyse eine in Dioxan mit methanolischer Natronlauge oder Kalilauge durchgeführte Alkoholyse ist.

**10.** Verbindungen der Formel (III):

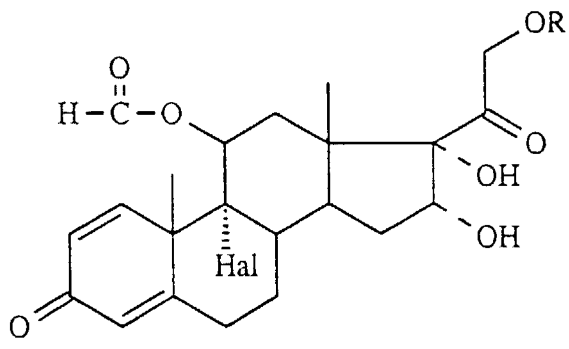

(III)

in der Hal und R die in Anspruch 1 angegebene Bedeutung besitzen, in ihrer Eigenschaft als neue industrielle Produkte.

**11.** Verbindungen der Formel (IV):

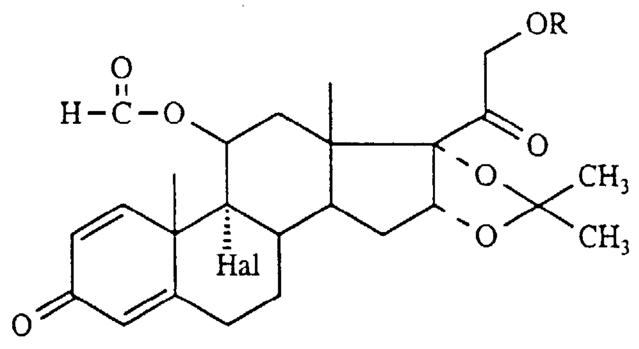

(IV)

in der Hal und R die in Anspruch 1 angegebene Bedeutung besitzen, in ihrer Eigenschaft als neue industrielle Produkte.

**12.** Verbindungen der Formel (I):

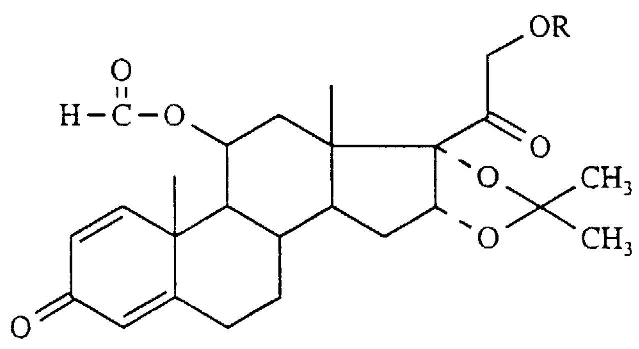

(I)

in der R einen Alkoxycarbonyl- oder Arylalkoxycarbonylrest mit 2 bis 8 Kohlenstoffatomen oder einen Alkyl- oder Arylsulfonylrest mit maximal 8 Kohlenstoffatomen darstellt, in ihrer Eigenschaft als neue industrielle Produkte.

**13.** Verbindungen der Formel (VI):

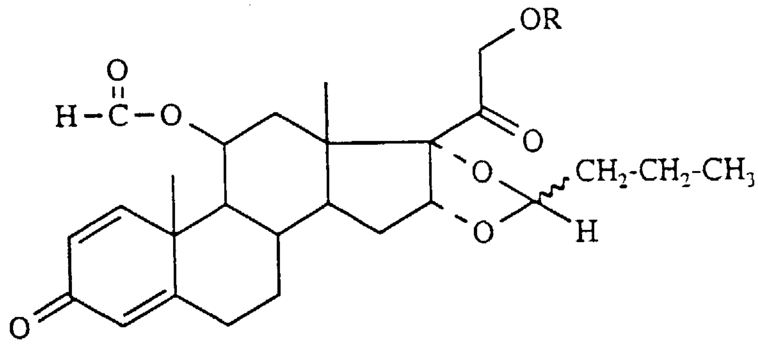

(VI)

in der R und die wellenförmig dargestellte Bindung die in Anspruch 1 angegebene Bedeutung besitzen, in ihrer Eigenschaft als neue industrielle Produkte.

**14.** Verbindungen der Formel (VII):

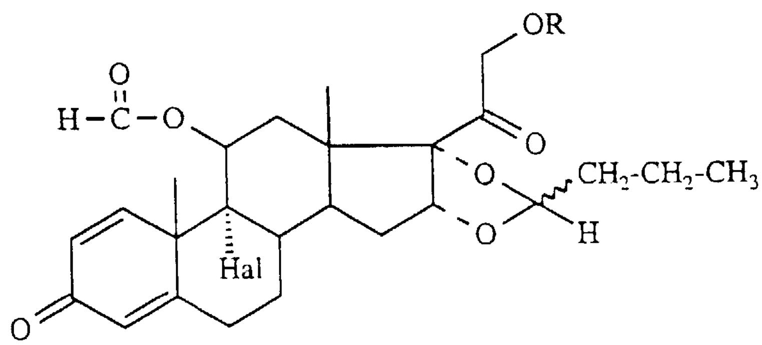

(VII)

in der Hal, R und die wellenförmig dargestellte Bindung die in Anspruch 1 angegebene Bedeutung besitzen, in ihrer Eigenschaft als neue industrielle Produkte.

**Claims**

**1.** Process for the preparation of the compound of formula (V):

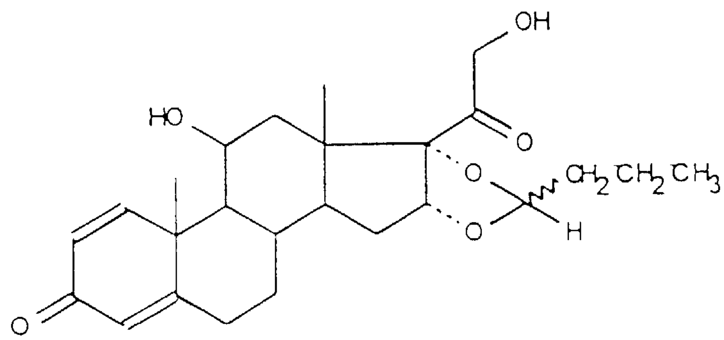

(V)

in which the wavy line indicates the existence of two isomers, the compound appearing in the form of a mixture of these isomers, characterized in that a compound of general formula (II):

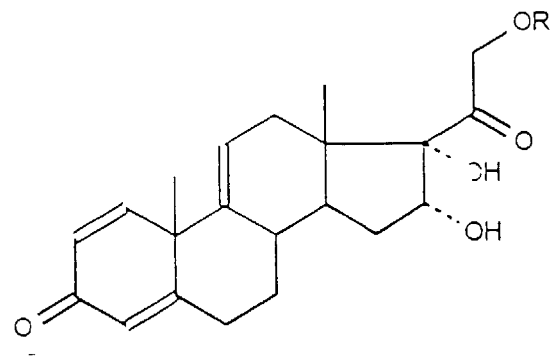

(II)

in which R represents a hydrogen atom, an acyl radical containing 1 to 8 carbon atoms, an alkoxycarbonyl or aralkoxycarbonyl radical containing 2 to 8 carbon atoms or an alkyl or aryl sulphonyl radical containing up to 8 carbon atoms, is treated by a chlorination or bromination agent and a formyloxylation agent, in an acid medium, in order to obtain a compound of general formula (III):

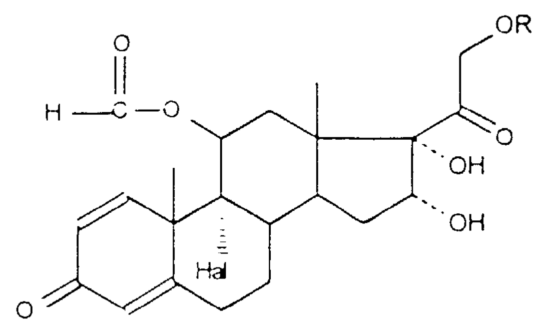

(III)

in which R is defined as previously and Hal represents a chlorine or bromine atom, which is treated by acetone in the presence of a strong acid, in order to obtain a compound of general formula (IV):

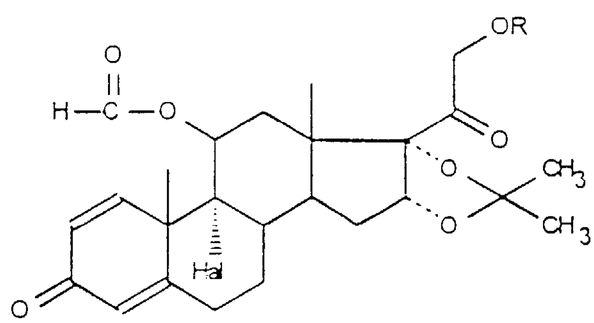

(IV)

which is treated by a dehalogenation agent, in order to obtain a compound of general formula (I):

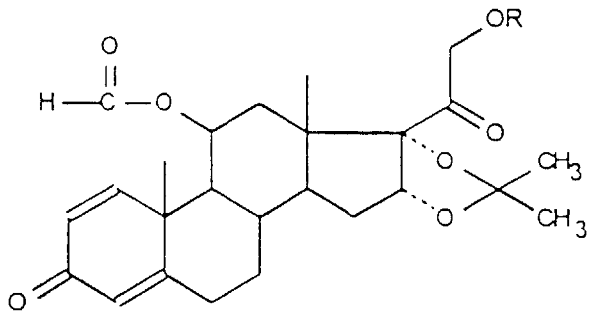

(I)

in which R is defined as previously, which is treated by butanal in the presence of a strong acid catalyst, in order to obtain a compound of formula (VI):

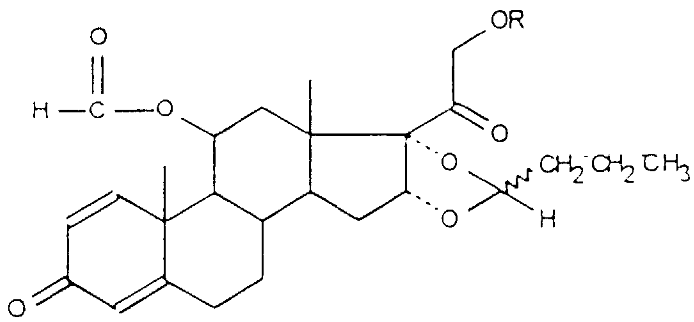

(VI)

in which R and the wavy line are defined as previously, which is subjected to a solvolysis in a basic medium, in order to obtain the expected compound of formula (V).

**2.** Process according to claim 1, characterized in that R represents an acetyl radical.

**3.** Use according to claim 1 or 2, characterized in that:

- the chlorination or bromination agent is chosen from the group constituted by N-chloro and N-bromo acetamides, succinimides and phthalimides, N,N-dichloro and N,N-dibromo dimethyl hydantoins, tert-butyl hypochlorite and tert-butyl hypobromite;
- the formyloxylation agent is dimethylformamide or formic acid;
- the strong acid used during ketalization is perchloric acid or fluoroboric acid;
- the ketalization is carried out in the presence of a dehydrating agent;
- the dehalogenation is carried out by the action of tributyltin hydride, in the presence of a radical reaction initiator;
- the strong acid catalyst is perchloric acid;
- the final solvolysis is an alcoholysis carried out using methanolic soda or potash, in dioxane.

**4.** Process for the preparation of the compound of formula (V):

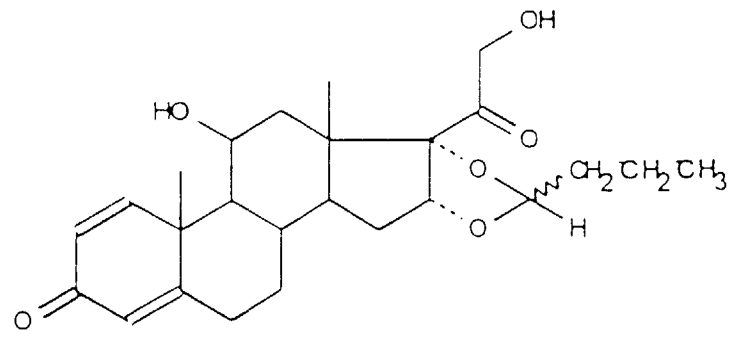

(V)

in which the wavy line indicates the existence of two isomers, the compound appearing in the form of a mixture of these isomers, characterized in that a compound of general formula (II):

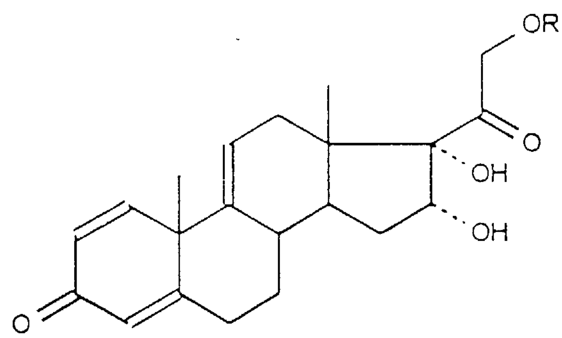

(II)

in which R represents a hydrogen atom, an acyl radical containing 1 to 8 carbon atoms, an alkoxycarbonyl or aralkoxycarbonyl radical containing 2 to 8 carbon atoms or an alkyl or aryl sulphonyl radical containing at most 8 carbon atoms, is treated by a chlorination or bromination agent and a formyloxylation agent, in an acid medium, in order to obtain a compound of general formula (III):

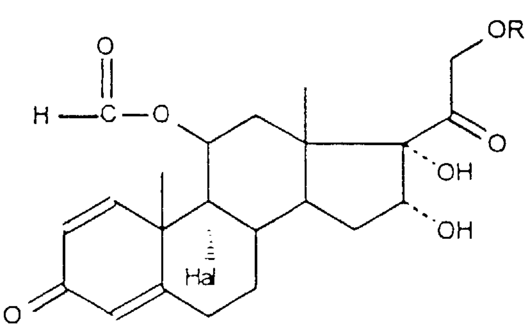

(III)

in which R is defined as previously and Hal represents a chlorine or bromine atom, which is treated by acetone in the presence of a strong acid, in order to obtain a compound of general formula (IV):

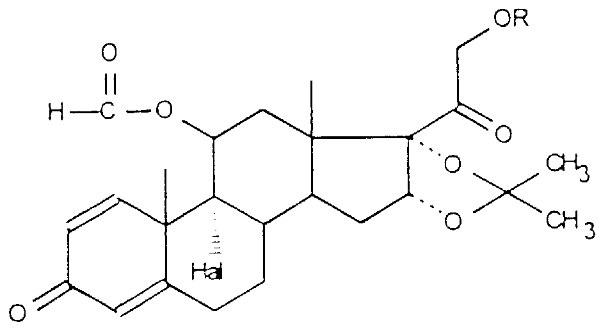

(IV)

which is treated by butanal in the presence of a strong acid catalyst, in order to obtain a compound of formula (VII):

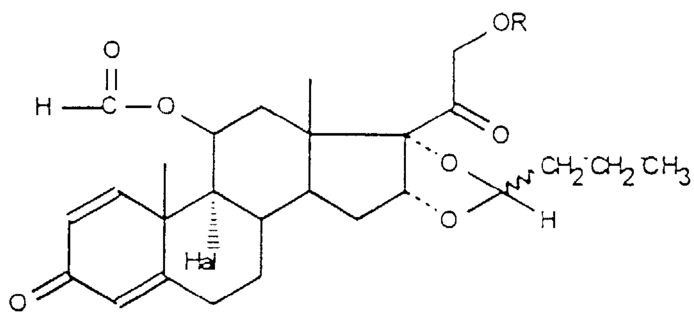

(VII)

in which Hal, R and the wavy line are as defined previously, which is treated by a dehalogenation agent in order to obtain a compound of formula (VI):

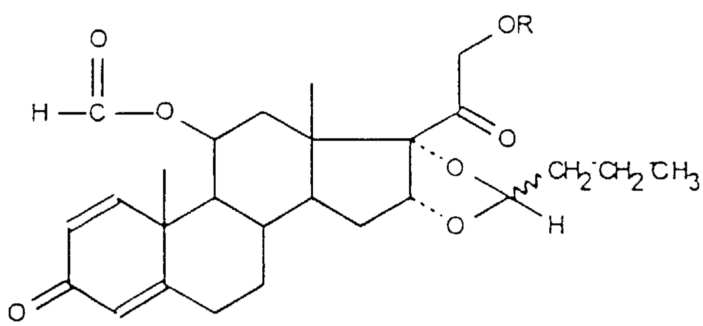

(VI)

in which R and the wavy line are defined as previously, which is subjected to a solvolysis in a basic medium, in order to obtain the expected compound of formula (V).

**5.** Process according to claim 4, characterized in that R represents an acetyl radical.

**6.** Process according to claim 4 or 5, characterized in that:

- the chlorination or bromination agent is chosen from the group constituted by N-chloro and N-bromo acetamides, succinimides and phthalimides, N,N-dichloro and N,N-dibromo dimethyl hydantoins, tert-butyl hypochlorite and tert-butyl hypobromite;
- the formyloxylation agent is dimethylformamide or formic acid;
- the strong acid used during ketalization is perchloric acid or fluoroboric acid;
- the ketalization is carried out in the presence of a dehydrating agent;
- the strong acid catalyst is perchloric acid;

- the dehalogenation is carried out by the action of tributyltin hydride, in the presence of a radical reaction initiator;
- the final solvolysis is an alcoholysis carried out using methanolic soda or potash, in dioxane.

**7.** Process for the preparation of the compound of formula (VIII):

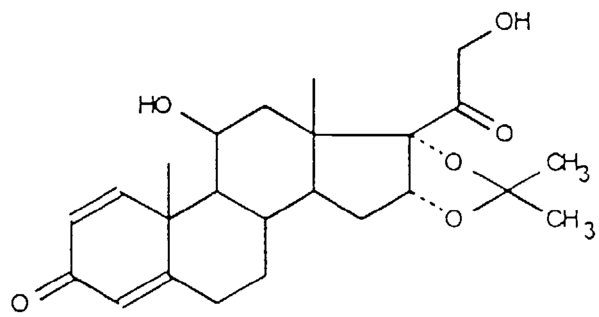

(VIII)

characterized in that a compound of general formula (II):

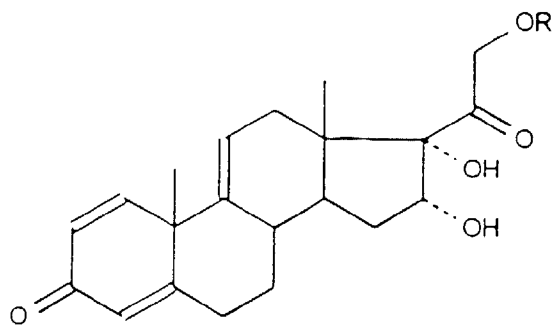

(II)

in which R represents a hydrogen atom, an acyl radical containing 1 to 8 carbon atoms, an alkoxycarbonyl or aralkoxycarbonyl radical containing 2 to 8 carbon atoms or an alkyl or aryl sulphonyl radical containing up to 8 carbon atoms, is treated by a chlorination or bromination agent and a formyloxylation agent, in an acid medium, in order to obtain a compound of general formula (III):

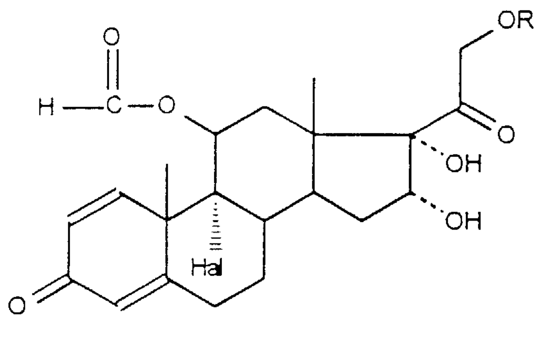

(III)

in which R is defined as previously and Hal represents a chlorine or bromine atom, which is treated by acetone in the presence of a strong acid, in order to obtain a compound of general formula (IV):

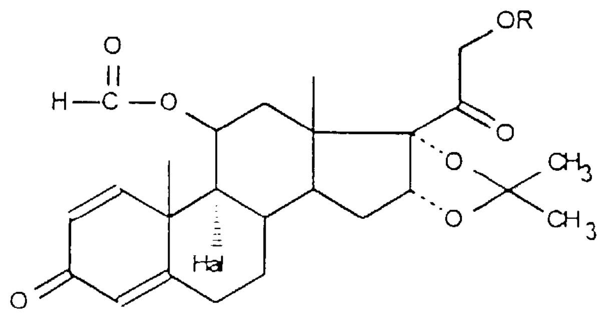

(IV)

which is treated by a dehalogenation agent, in order to obtain a compound of general formula (I):

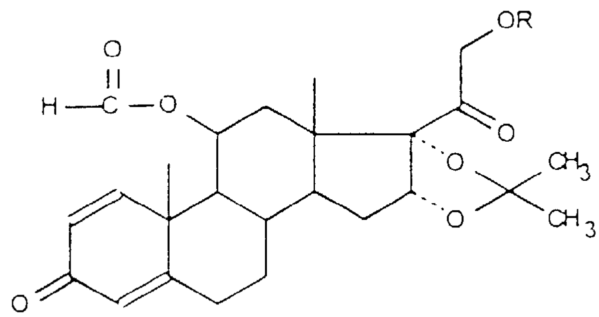

(I)

in which R is defined as previously, which is subjected to a solvolysis in a basic medium, in order to obtain the expected compound of formula (VIII).

**8.** Process according to claim 7, characterized in that R represents an acetyl radical.

**9.** Use according to claim 7 or 8, characterized in that:

- the chlorination or bromination agent is chosen from the group constituted by N-chloro and N-bromo acetamides, succinimides and phthalimides, N,N-dichloro and N,N-dibromo dimethyl hydantoins, tert-butyl hypochlorite and tert-butyl hypobromite;
- the formyloxylation agent is dimethylformamide or formic acid;
- the strong acid used during ketalization is perchloric acid or fluoroboric acid;
- the ketalization is carried out in the presence of a dehydrating agent;
- the dehalogenation is carried out by the action of tributyltin hydride, in the presence of a radical reaction initiator;
- the final solvolysis is an alcoholysis carried out using methanolic soda or potash, in dioxane.

**10.** As new industrial compounds, the compounds of formula (III):

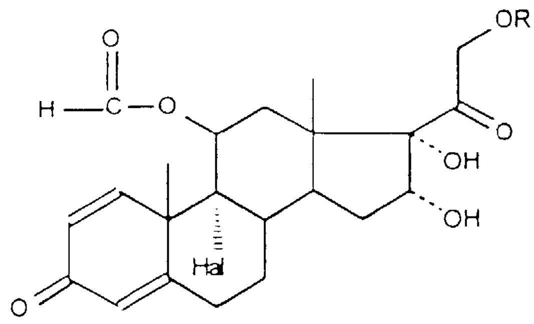

(III)

in which Hal and R are defined as in claim 1.

**11.** As new industrial compounds, the compounds of formula (IV):

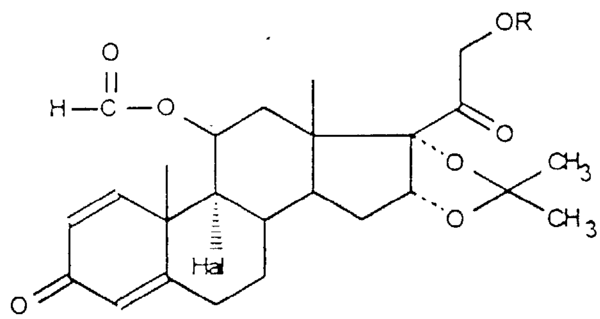

(IV)

in which Hal and R are defined as in claim 1.

**12.** As new industrial compounds, the compounds of formula (I):

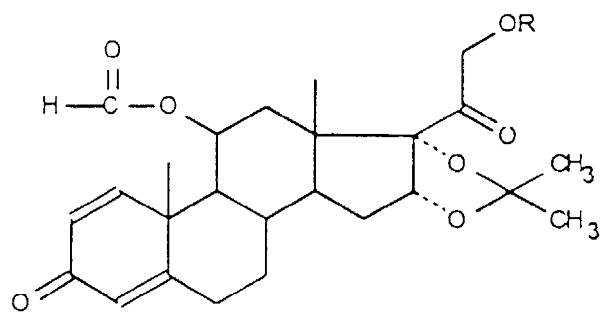

(I)

in which R represents an alkoxycarbonyl or aralkoxycarbonyl radical containing 2 to 8 carbon atoms or an alkyl or aryl sulphonyl radical containing up to 8 carbon atoms

**13.** As new industrial compounds, the compounds of formula (VI):

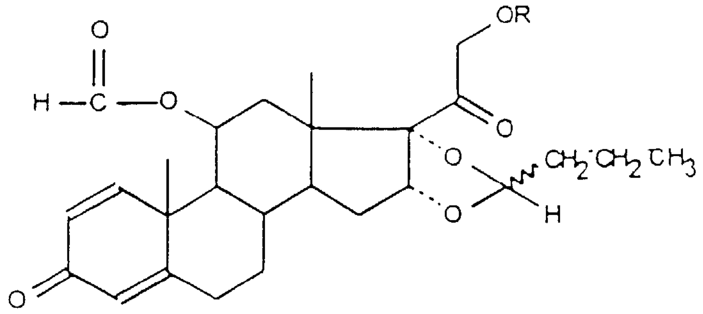

(VI)

in which R and the wavy line are defined as in claim 1.

**14.** As new industrial compounds, the compounds of formula (VII):

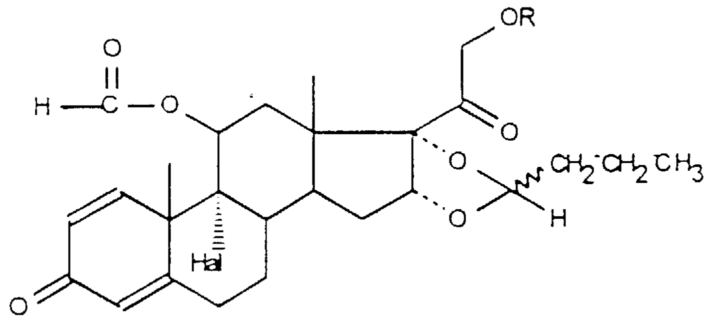

(VII)

in which Hal and R and the wavy line are defined as in claim 1.